# EUROPEAN PATENT APPLICATION

(11) **EP 2 277 441 A1**
(43) Date of publication of application: **26.01.2011**
(21) Application number: 10170547.3
(22) Date of filing: 22.07.2010
(51) Int. Cl.: A61B 5/05

(54) **Method for generating images of a human body zone undergoing a surgical operation by means of an apparatus for minimally invasive surgical procedures**

(30) Priority: 22.07.2009 IT BO20090470
(71) Applicant: Surgica Robotica S.p.A., Trieste (IT)
(72) Inventor: Fiorini, Paolo, 37134 Verona (IT); Botturi, Debora, 37160 Castel D'azzano (IT)
(74) Representative: Jorio, Paolo

(57) **Abstract**

An apparatus for minimally invasive surgical procedures comprising a first surgical robot device (21) provided with a respective surgical instrument (12), a haptic device (11) with force feedback for allowing an operator to control the first surgical robot device (21), a second surgical robot device (12) provided with an ultrasound sensor (22) for acquiring data which allows a direct view of the operation zone, a processing unit (60), and several software objects loadable on the processing unit (60) and designed to create a three-dimensional model of the operation zone on the basis of pre-surgery images, for acquiring markers of the three-dimensional model, for updating the position of markers in real time and on the basis of data acquired by means of the ultrasound sensor (22), for updating the three-dimensional model as a function of the updated position of markers and for generating images of the operation zone on the basis of the updated three-dimensional model.

## Description

The present invention relates to a method for generating images of a human body zone undergoing a surgical operation by means of an apparatus for minimally invasive surgical procedures and to a corresponding apparatus for minimally invasive surgical procedures.

Apparatuses for surgical procedures, in particular of the minimally invasive type, which use control, simulation and planning systems are known. These apparatuses intend to guide the various operation steps, generally providing the operator with representative images of more or less wide areas of the surgical operation zone, obtained either in a pre-surgery step or during the operation itself.

The images obtained in a pre-surgery step may be, for example, acquired by means of known acquisition methods, e.g. by means of computerized tomography (TC) or by means of magnetic resonance imaging (MRI). These images are usually used to process three-dimensional representations of the anatomic areas involved in the surgical operation to be performed. The so-processed representations have the purpose of allowing the surgeon to plan the various steps of the surgical procedure to be performed.

In contrast, the images obtained during surgery, the so-called "intra-surgery" images, may be acquired in a known manner by means of devices of the intra-surgical camera or ultrasound scanner type, or by means of fluoroscopy and magnetic resonance performed during the surgical procedure.

For example, patent application US 2008/0234700 describes a method and apparatus for planning the path of an endoscopic instrument, such as for example a catheter, in a patient's bronchi. The instrument is controlled in a three-dimensional calculation environment by taking the freedom degrees of the instrument and the patient's morphology into account. The result of such a calculation is used to plan and simulate the optimal path of the instrument, but also to train medical staff, to compare various surgical techniques or to automate surgical procedures. The method further allows to indicate the optimal instrument for performing the specific surgical procedure with reference to shape and flexibility thereof, but also to the patient's particular morphology. Finally, the method allows to calculate the kinematically possible path between an initial configuration and a final configuration of the instrument, avoiding possible obstacles or dangerous regions which may be identified in the patient's anatomy.

Patent application US 2008/0065109 describes a method for avoiding possible collisions between minimally invasive instruments and the patient's tissues. The method includes mapping an anatomic cavity of the patient by means of the recordings made by a first surgical instrument. Such a mapping is used by a second surgical instrument subsequently introduced. The method further includes defining a volume within which a control system allows the instrument to move. In particular, such a volume is defined by the sum of a first volume constructed according to possible movements of the instrument and of a further volume constructed according to the visual contours of a component capable of picking up images. The real-time viewing of a surgical environment is associated in a mosaic with the viewing of a previously recorded image related to a distal zone of the same environment. The surgical instruments are made visible in real time viewing, while virtual representations of the same instruments are generated and viewed on the mentioned recorded image at the same time. This virtual view provides a representation of the instruments outside the viewing field of the system which detects images in real time.

Other planning methods are known, which rely on the simulation of operations guided by viewing the anatomic operation zone, as well as methods for implementing surgical procedures guided by images.

The known methods do not however allow to meet the reliability requirements of today's surgical procedures, in particular of minimally invasive surgical procedures. Indeed, the known apparatuses and guiding methods do not allow to simulate the operation environment as really modified by the interaction with surgical instruments, thus rendering instead simulations obtained from pre-surgery data or, in all cases, from data not updated in real time. These limitations thus create considerable doubts about the efficacy of the aforesaid apparatuses and of the corresponding guiding methods, furthermore excluding the opportunity of using them in a fully automatic mode.

It is the object of the present invention to provide a method for generating images of a patient's body zone intended to be subjected to or undergoing a surgical operation by means of an apparatus for minimally invasive surgical procedures, and to provide a coresponding apparatus for surgical procedures, which are free from the above-described drawbacks while being easy and cost-effective to be implemented.

In accordance with the present invention, a method for generating images of a patient's body zone intended to be subjected to a surgical operation and an apparatus for minimally invasive surgical procedures are provided as defined in the appended claims.

The present invention will now be described with reference to the accompanying drawings, which illustrate a non-limitative embodiment thereof, in which:
- figure 1 diagrammatically shows the apparatus for minimally invasive surgical procedures made according to the dictates of the present invention; and
- figure 2 shows a flow chart of the image generating method provided with the invention, which method is implemented by the apparatus of figure 1.

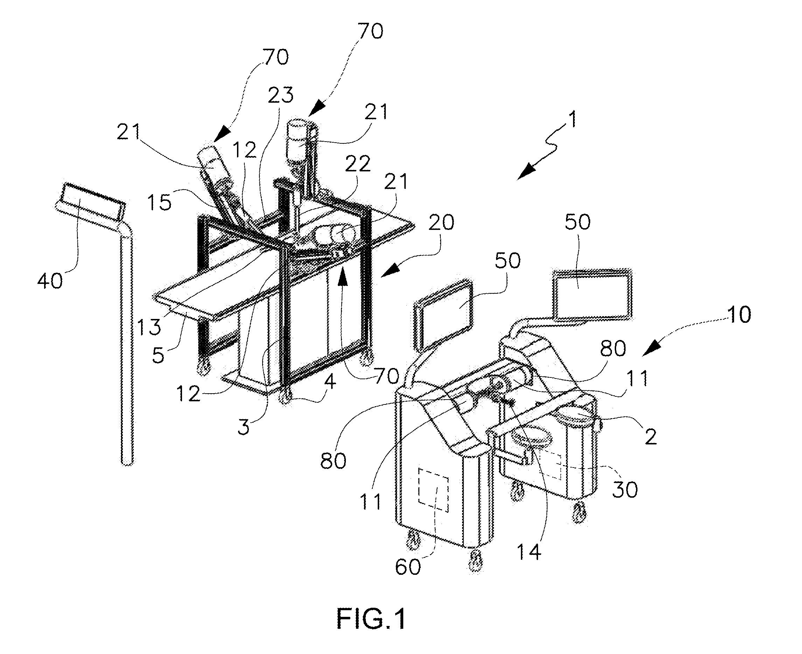

In figure 1, reference numeral 1 generally indicates as a whole an apparatus for minimally invasive surgical procedures provided according to the invention. Apparatus 1 comprises a master station 10, preferably trolley-mounted and provided with one or more seats 2 for one or more operators. Master station 10 comprises at least one haptic device 11 with force feedback of the known type, preferably consisting of a force feedback joystick, i.e. a joystick capable of reproducing the forces generated by the interaction between the surgical instruments 12 and the zone undergoing surgery in a manner perceivable by the operator. Apparatus 1 preferably comprises two haptic devices 11, as shown in figure 1. Each haptic device 11 has at least six degrees of freedom, preferably but not necessarily comprises force sensors, and has an ergonomic handle 14 for allowing the surgeon to control the surgical instruments 12 with skill.

Apparatus 1 comprises at least one slave station 20, comprising at least two surgical robot devices 21 each having at least four degrees of freedom. In the embodiment shown in figure 1, the surgical robot devices 21 are carried by a movable supporting trolley 3 on wheels 4 having a bridge structure to be arranged over a surgical bed 5 of the known type on which the patient is laid. Each surgical robot device 21 comprises a respective robotized articulated arm. In at least a first surgical robot device 21, the articulated arm carries and moves a surgical instrument 12 adapted to be inserted into the patient's body to perform minimally invasive surgical procedures on the patient's body. In a second surgical robot device 21, the articulated arm carries detection means 22 adapted to be inserted into the patent's body to directly detect data related to the body zone intended to be subjected to or undergoing a surgical operation in real time for the purpose of allowing direct, real view of such a body zone, e.g. during the surgical operation. Figure 1 shows two surgical robot devices 12, each provided with a respective surgical instrument 12. The surgical robot devices 12 which carry the surgical instruments 12 may be controlled by the operator by means of the haptic devices 11.

The surgical robot devices 21 may be positioned, in use, close to the patient. The position of the surgical robot devices 21 is adjustable along guiding crosspieces 23 of trolley 2 by means of a low-precision positioning device, either manually or remotely actuated by the surgeon, in order to separate high-precision functions, specific of the surgical robot device 21, from low-precision functions, which may be performed by the mentioned positioning device.

The surgical robot devices 21 are actuated by motor members, preferably placed apart from the articulated joints of the respective robotized articulated arm. In particular, the motor members are arranged at one end of the articulated arm, mounted to trolley 3, and command the respective articulated joints by means of cables. The surgical instruments 12 are mounted to the opposite end of the articulated arm by means of a wrist 13 with three degrees of freedom, which is capable of performing two rotations with respect to two perpendicular axes and one rotation about a main axis of instrument 12, and comprising the mechanics for transferring the opening and closing movements to the surgical instrument 12, and are adapted to be inserted into the patient's body to perform the desired surgical procedures. The movements of each surgical instrument 12 are imparted by some motor members of the respective robotized articulated arm. A rapid replacement device 15 for the surgical instruments 12 is further provided for releasably connecting the surgical instrument to the wrist 13 of the articulated arm of a surgical robot device 21.

The detection means 22 are of the optical and/or ultrasound type, and particularly comprise a camera and at least one ultrasound sensor consisting, for example, of a sonographic probe or, more specifically, a laparoscopic ultrasound probe.

Apparatus 1 comprises one or more high-performance, computational processing units 60 arranged in master station 10, control and actuating means 70 arranged in slave station 20 for actuating the surgical robot devices 21, and control and actuating means 80 arranged in master station 10 for controlling the devices of master station 10, including the haptic devices 11. Apparatus 1 further comprises a storage unit 30 associated with the processing unit 60 to store data related to the patient's body intended to be subjected to a surgical operation, and software means which are stored in the storage unit 30, are loadable by the processing unit 60 and are designed to process data related to the patient's body, in the manner described hereinafter, when run on the processing unit 60.

The software means of apparatus 1 comprise simulation means, which comprise at least one software object, indicated hereinafter by SWM, for simulating the patent's anatomy. In particular, the software object SWM allows to create a three-dimensional, graphical-physical model of the zone intended to be subjected to the operation starting from pre-surgery images of the zone intended to be subjected to surgery. The software object SWM creates such a three-dimensional model on the basis of a sophisticated mathematical model and by using segmentation and modeling algorithms. The mathematical model is calibrated on the mechanical features of the organs present in the patient's body zone intended to be subjected to the surgical operation. The aforesaid pre-surgery images area obtained, for example, by pre-surgery detection means of the computerized axial tomography (CAT) or of the magnetic resonance imaging (MRI) type. More in detail, the software object SWM creates a three-dimensional graphic model by processing the pre-surgery images and attributes given physical properties to the graphic model by means of the aforesaid mathematical model so as to obtain a three-dimensional, graphical-physical model, such as to allow to calculate interaction forces between the model itself with the surgical instruments 12, when the latter are arranged, either really or in the step of simulating the surgical procedure, in the operation zone.

Furthermore, the simulation means comprise at least another software object, indicated hereinafter by SWS, which allows to simulate a surgical procedure on the previously created three-dimensional, graphic-physical model in the absence of the patient's body, as will be explained in greater detail below. The software object SWS allows to simulate the surgical procedures on the patient's virtual anatomy by using the three-dimensional model of the operation zone and the models of the surgical instruments 12, and by acquiring commands from the haptic devices 11. In the operation zone, the interactions between the virtual instruments and the models of the patient's organs are simulated, the modifications of the operation zone as a function of the aforesaid interactions are determined, and the three-dimensional mode is updated in real time on the basis of the modifications of the operation zone in the simulated surgical procedure. The simulated interactions comprise contact and changes in topology, such as for example cuts and biopsies. Furthermore, the feedback forces to be transmitted to the haptic means 11 are generated as a function of the simulated contacts and changes in topology.

The software means of apparatus 1 preferably comprise, either in addition to or incorporated in the simulation means, planning means for planning the surgical operation during the pre-surgery step. The planning means comprise at least another software object, indicated hereinafter by SWP, which allows, once run on the processing unit 60, to plan the real surgical procedure which will be performed on the patient during the simulation of the surgical procedure. The pre-surgery planning of the operation aids the surgeon during the real surgical procedure, by providing information on the contact forces and on the path that the instruments 12 must follow inside the patent's body. Planning the surgical procedure implies the software object SWS to be run being combined with the software object SWP. Once the surgeon is satisfied with the development of the simulated surgical procedure, the latter may be saved in storage unit 30. In particular, planning a surgical procedure consists in either selecting or generating optimal paths for the surgical instruments 12 during the simulation of the surgical procedure, in generating appropriate constraints and suitable warning signals with reference to the patient's real anatomy, and in saving the whole in the storage unit 30. The optimal paths of the instruments may be either chosen by the surgeon or autonomously calculated by the planning means without the surgeon's direct intervention, during the simulated surgical procedure.

Apparatus 1 comprises recording or regulation means for matching the three-dimensional model of the operation zone created by the software object SWM, with images or data related to the real anatomy of the patient undergoing the operation. Such a matching is based on the identification of markers, preferably both outside and inside the patient, the position of which is used as a reference for updating the three-dimensional model. In other words, updating the three-dimensional model results from the recording or regulation of the three-dimensional graphic model with the patients' real anatomy, in particular with the real position of the mentioned markers.

The recording means comprise, in particular, a recording instrument 40 consisting, for example, of a stereoscopic camera of known type for recording the three-dimensional model with real external anatomy of the patient's body laying on bed 5 just before starting the surgical procedure. In particular, such a recording includes selecting appropriate markers outside the patient's body by applying appropriate labels on the body, calculating their absolute position in space and correcting the "outermost" part of the three-dimensional model with the calculated positions. Alternatively, such a recording may be carried out without the calibration instrument 40 and by appropriately controlling a surgical robot device 21 fitting a camera. The purpose of this "external" recording is to correct, although approximately, the three-dimensional model with the position of the internal organs and the deformations associated with the real position of the patient's body.

The recording means further comprise a software object SWU, which generally belongs to the software means of apparatus 1, and allows to acquire data by means of the detection means 22, and in particular by means of the ultrasound sensor, and to update the position of a cloud of markers within the three-dimensional model as a function of the acquired data. This "internal" recording allows to correct the three-dimensional model in real time during the surgical procedure as a function of real deformations sustained by the zone undergoing the operation, in particular those sustained by the organs due to the contact with the surgical instruments 12, the biological movements and the gravity action.

Apparatus 1 further comprises three-dimensional viewing means 50 for viewing detailed three-dimensional images of the operation zone. The viewing means 50 comprise one or more displays controlled by at least one high-performance graphic board for viewing the images of the operation zone with precision real time. These images are either generated only on the basis of real data of the operation zone acquired directly by means of the detection means 22, or are generated on the basis of a virtual reality representing the operation zone, i.e. only on the basis of the graphic three-dimensional model, or are images resulting from the real-time matching of the three-dimensional model of the patient's anatomy with real data of the operation zone in a so-called "augmented reality" mode. The continuous (real time) matching of three-dimensional model and real data of the operation zone allows to obtain a high level of precision and also comprises areas which are not directly detectable by the detection means 22.

Apparatus 1 for minimally invasive surgical procedures may therefore be used for obtaining and viewing a continuous data flow (in real time) representing the operation zone both during the pre-surgery step, i.e. during the step of simulating and planning of a surgical procedure, and during the real execution of a surgical procedure.

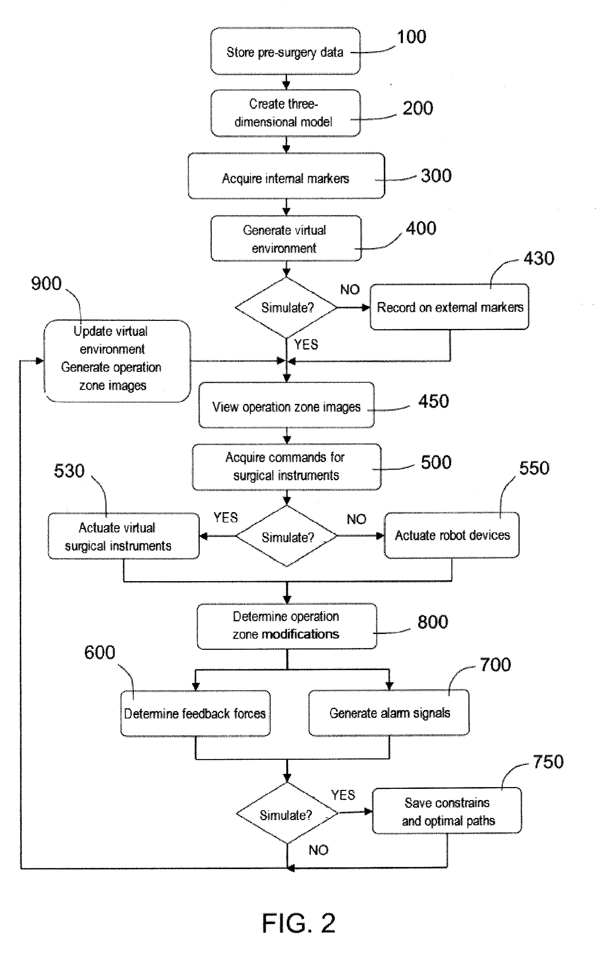

The processing unit 60 is configured to run apparatus 1 as shown in the flow chart in figure 2. In particular, the software means of apparatus 1 comprise a main software object SWMAIN designed to coordinate the software objects SWM, SWS, SWP and SWU so as to implement the image generating method of the invention when performed on the processing unit 60, which method comprises the steps of the flow chart shown in figure 2.

With reference to figure 2, in a first step of preparing the desired surgical procedure, the operator, for example the surgeon or the radiologist, prepares pre-surgical data related to images of the operation zone, such as, for example, computerized axial tomography (CAT) or magnetic resonance imaging (MRI). This pre-surgical data is stored in the storage unit 30 of the master station 10 (block 100). The three-dimensional model of the zone to be subjected to surgery is created with the aid of the software object SWM (block 200). One or more markers of the three-dimensional model are acquired after having created the three-dimensional model (block 300). Optionally, data is further acquired to mark prohibited anatomical areas, i.e. areas which must not be touched during the operation, or risky anatomic areas. The markers and the prohibited or risky areas are selected and entered into the processing unit 60 by the operator by means of a man-machine interface, e.g. by means of a keyboard. The markers will be used for subsequent updates of the three-dimensional model during the simulation and/or execution of the surgical procedure. Furthermore, the three-dimensional model may be used both for the purpose of analysis or preliminary study and for preparing a given surgical procedure to be performed.

At this point, apparatus 1 is ready either to simulate the surgical procedure chosen on the three-dimensional model, dwelling on the various surgical steps and storing the virtually performed sequence of actions in the processing unit 60, or for performing the surgical procedure in practice. Normally, the execution of the surgical procedure is preceded by a simulation of the same. However, in either case, with the aid of the software object SWS, a virtual environment, which comprises the three-dimensional model of the zone undergoing surgery, is generated, which is interactive, meaning that it may interact with the commands from the haptic devices 11 (block 400). The virtual environment further comprises virtual surgical instruments, which are created on the basis of the mechanical models of the respective surgical instruments 12. Images of the operation zone are further generated during the step of generating of the virtual environment.

The previously generated images of the operation zone are viewed during the simulation of the surgical procedure (block 450). The movement commands that the operator intends to supply to the surgical instruments 12 are acquired by means of the haptic devices 11, which are handled by the operator (block 500). With the aid of the software object SWS, the virtual surgical instruments are actuated as a function of the movement commands acquired by means of the haptic devices 11 (block 530).

With the aid of the software object SWS, the interactions between the zone intended to be subjected to the operation and the virtual surgical instruments are determined and the position of the internal markers are updated as a function of such interactions (block 800). In particular, morphological and/or topographical modifications of the operation zone and displacements of the virtual surgical instruments are calculated on the basis of the three-dimensional model and to the commands acquired by means of the haptic devices 11. The new position of the internal markers is calculated as a function of these modifications.

Again with the aid of the software object SWS, the feedback forces to be transmitted to the haptic devices 11 are calculated on the basis of the previously calculated modifications of the operation zone and displacements of the virtual surgical instruments (block 600). At the same time, appropriate visual alarm signals by means of viewing means 50, and/or acoustic signals, and/or haptic signals by means of the haptic devices 11 are generated for signaling possible risky situations, such as for example the virtual collision of the virtual surgical instruments or the proximity to prohibited or risky areas (block 700). In particular, the visual alarm signals consist of, for example, graphic icons viewed on viewing means 50, and the haptic signals consist of feedback forces such as to hinder the movement of the haptic devices 11 by the operator.

With the aid of the software object SWP, further constraints referred to the patient's anatomy and respective alarm signals, or optimal paths for the instruments 12 and/or single sub-steps of the simulated surgical procedure may be saved, when requested by the operator, in the storage unit 30 of the master station 10 (block 750). In this manner, the simulation of the surgical procedure may be later used as guide in the real surgical procedure.

Finally, the three-dimensional model, and more in general the virtual environment, is updated by matching in real time the three-dimensional model itself with the new position of the markers and the new images of the operation zone are generated on the basis of the updated three-dimensional model (block 900). The virtual environment and of the images of the operation zone are updated by means of the software object SWS. The viewing of the new images of the surgical operation zone is updated in real time (block 450).

During the real surgical procedure, apparatus 1 works by substantially following the steps described above, with the following differences.

Once the virtual environment has been created, the three-dimensional model is recorded with the markers outside the patient's body by means of the recording instrument 40, as previously described (block 430). The three-dimensional model recorded on the basis of the external markers is then updated in real time with the new position of the internal markers (block 900).

The operator's commands acquired by means of the haptic devices 11 are sent to the surgical robot devices 21 so that the latter are actuated as a function of these commands (block 550). Upon the operator's request, the surgical robot devices 21 may be acquired as a function of a matching of the commands acquired by the surgical robot devices 21 with the constraints and the optimal paths previously saved by the planning means during the simulation of the surgical procedure.

The interactions between the zone undergoing the operation and the surgical instruments 12 are determined with the aid of the software object SWU, i.e. on the basis of the data acquired by means of the detection means 22, and in particular by means of the ultrasound sensor and by means of the force sensors, if present, associated with the haptic devices 11 (block 800). In particular, the new position of the markers and the displacements of the surgical instruments 12 are determined in real time as a function of the data acquired by the detection means 22. The real time processing allows to signal possible risky situations, such as, for example, the collision of the surgical instruments 12, the proximity to a prohibited or risky area and the possible departure from the optimal path determined during the simulation (block 700).

The feedback forces to be transmitted to the haptic devices 11 are either measured directly as a function of the data acquired by the force sensors associated with the haptic devices 11 or as a function of the position and/or orientation error between the haptic devices 11 and the surgical instruments 12 (block 600). Such a position and orientation error may be calculated as a function of the displacements of the surgical instruments 12 determined in step 800. Such a calculation may be calculated by the main software object SWMAIN or by a specific software object SWF recalled by the software object SWMAIN.

Thus, the real surgical procedure may benefit from images of the operation zone (block 450) based on the continuous matching of the three-dimensional model created by means of the simulation with real data acquired by means of the detection means 22. Indeed, during the execution of the real surgical procedure, the position of the markers is updated in real time as a function of the data acquired by means of the detection means 22 and the three-dimensional model is updated in real time with the new position of the markers. It is worth noting that the real data provided by the detection means 22 allow in themselves to construct low quality (low definition) images with high precision with regards to the position of the various members, while the three-dimensional model allows to construct high-quality images also representing areas which are not directly detectable by the detection means 22, e.g. blood vessels which are behind tissues which are detectable or visible by the detection means 22. In other words, the matching of the three-dimensional model with the real operation zone data allows to obtain a panoramic view of the operation zone which has high quality and high precision at the same time.

By way of non-limitative example, the description of a generic surgical procedure applicable to a plurality of specific cases, such as, for example, resection of tumors, enucleation, removal and many other laparoscopic procedures, is shown below.

The surgeon creates the three-dimensional, graphic-physical model starting from the pre-surgery images of the concerned zone to evaluate the patient's operability. The surgeon may thereof simulate a surgical procedure, starting from a visual inspection and virtual palpation of the zone intended to be subjected to surgery, using the three-dimensional viewing means 50 and the haptic devices 11. In particular, the three-dimensional model allows the surgeon to evaluate the consistency of tissues, the proximity to other anatomic structures and to evaluate the access paths to the target of the surgical operation.

Subsequently, the surgeon may determine, with the aid of the haptic devices 11, the access path to the target of the surgical operation, the type and the size of the surgical instrument 12 to be used, as well as the precision required for the safe execution of the desired surgical procedure.

During the simulation of the surgical procedure, the surgeon can further identify the risky areas and accordingly instruct the processing unit 60 to generate warning signals if the instrument 12 approaches the aforesaid areas.

Furthermore, the type of warning to be generated, as well as the distance from which the signal must be generated, may be specified for each type of warning signal, and a "virtual wall" may be defined to be adapted to generate a force feedback capable of blocking the movement of the surgeon's hand if the warning signal is not eliminated during the procedure.

During the simulation of the surgical procedure, the processing unit 60 may also store a plurality of warnings, adapted to indicate the possible approach to areas defined as critical in addition to the path chosen by the surgeon.

In order to use the data stored during the simulated surgical procedure, the pre-surgery images and the models of the patient's organs must be recorded on the patient's anatomy during the step of preparing and then of performing the desired surgical procedure. Such a recording occurs in the two operative steps described below.

An exterior recording is performed, adapting the three-dimensional model to the patient's real anatomic conformation so as to identify, although approximately, the position of the internal organs.

An internal recording is further performed, in which the simulated models of the organs initially allow to estimate the actual position of the patient's organs, by supplying the starting point for the precision recording of the organs to the detection means 22, e.g. an ultrasound scanner. The anatomic reference structures are identified in the images generated by the detection means 22 allowing to adapt the graphic model of the patient's anatomy to the real images, so that the simulation data corresponds to the patient's real anatomy.

The surgical procedure may be started at this point. The patient is prepared for the operation and insufflated. The trocars are inserted into the abdomen through which two or more surgical instruments 12 are preferably introduced, one for each trocar, a camera and a ultrasound probe. By means of the camera and the use of the haptic devices 11, the surgeon has the complete perception of the zone undergoing surgery and may start the operation.

The invention thus reaches the object of providing a method for generating images of a zone intended to be subjected toor undergoing a surgical operation which is capable of safety guiding the surgeon during a minimally invasive surgical operation. Such an object is obtained by integrating the direct viewing of the operation zone with a high-precision simulation of the operation zone. In particular, such a simulation is adapted to effectively guide the surgeon during the operation by virtue of the possibility of viewing both in detail and in panoramic mode the simulation of the operation zone. The simulated three-dimensional model is updated according to the data directly supplied by the detection means 22.

An important aspect of the method and corresponding apparatus described above is that a reliable simulation of the reality as effectively modified by the surgical instruments 12 is provided. Indeed, the newly created three-dimensional model, indicative of the reality not yet modified by the intervention of the surgical instruments or by the intra-surgical probes, is then updated at each step of calculating triggered by the data flow from the detection means 22.

An important aspect of the updating method of the three-dimensional model resides in the definition of the markers, which are substantially defined as movable entities with precision for the operation zone. The movements of the markers is continuously detected, monitored and calculated for updating the three-dimensional model, as well as the positioning of the surgical instrument in the operation zone during the simulation. In essence, the deformations and the current state of the three-dimensional model are obtained by superimposing the markers during the previous step of calculating with the current markers.

The simulation of the modified reality is furthermore very reliable, because the calculation of deformations takes into account not only the displacements of the surgical instruments actually used, but also the dynamic interactions involved, the observed biological movements and the properties of the concerned biological environment.

An important advantage further consists in that the method and apparatus described above allow to provide to the surgeon with a detailed, very accurate view of the zone in which the surgical procedure is performed, including the areas which are not directly visible of the operation zone. The simulation of the areas not directly visible is very important because the monitoring of the latter is needed for the purpose of continuance and success of the surgical procedure. The correct simulation of these areas indeed avoids the surgeon to continuously move the detection means 22 for exploratory purposes, thus minimizing the invasive impact of the surgical activity, the risks of damaging tissues and the total execution times of the surgical procedures.

It is worth noting that during the execution of the surgical procedure, the combined use of detection means 22, e.g. of the ultrasound scanner, and of the surgical robot device 21 allows to exactly evaluate the biomechanical parameters of the concerned organ or organs and thus to correct the anatomical models and the simulations of the respective displacements or deformations in real time.

Furthermore, by virtue of the continuous updating of the recording between the graphic model of the patient's organs and the real anatomy, the ideal path defined by the surgeon during the step of planning may be viewed on the patient's images and the warning signals may be generated in the correct positions.

By virtue of the use of haptic devices 11, and in particular of high-precision joysticks, and of the detection means 22, and in particular of a camera, and of the creation of a virtual reality, the surgeon can be easily and safely move the surgical devices 21 on which the surgical instruments 12 are positioned and may have a complete perception of the surgical operation, like in open surgery. By means of the articulation of the wrist 13 of the surgical instruments 12, the surgeon may easily perform the maneuvers needed for suturing tissue.

Furthermore, by virtue of the possibility of simulating the surgical procedure, apparatus 1 allows to process the virtual guiding signals, preferably in form of forces perceived by the operator at the joysticks. These signals are adapted to predict risky anatomical zones, e.g. by activating forces to oppose the movements of the control means which could be dangerous for the patient. The same signals may be used to predict and avoid the collision with other surgical instruments.

The method and apparatus provided with the invention thus allow to provide a sort of very reliable surgical navigator both during the step of preparing and the steps of performing the desired surgical procedure.

It is worth noting that the apparatus and method described may be used not only during the step of performing but also during the step of preparing, studying and even training for surgical procedures.

In the practical embodiment of the invention, the materials employed, as well as the shape and size thereof, may be of any type as needed.

Where the technical features mentioned in each claim are followed by reference marks, these reference marks are included for the sole purpose of improving comprehension of the claims and therefore they have no restrictive value on the purpose of each element identified by way of example by these reference marks.

## Claims

1. A method for generating images of a patient's body zone intended to be subjected to a surgical operation by means of an apparatus for minimally invasive surgical procedures, the apparatus (1) comprising at least a first surgical robot (21) provided with a respective surgical instrument (12), at least one haptic device (11) with force feedback for allowing an operator to control said first surgical robot device (21), and storage means (30); the method comprising:
- storing (100), in said storage means (30), first data related to pre-surgery images of the operation zone;
- creating (200) a three-dimensional, graphical-physical model of the operation zone on the basis of said first data and of a mathematical model calibrated on the mechanical features of the organs of the operation zone;
- acquiring (300), by means of man-machine interface means, first markers of said three-dimensional model;
- acquiring (500), by means of said haptic device (11), movement commands that the operator intends to supply to said first surgical robot (21);
- determining (800), in real time, interactions between said operation zone and said surgical instrument (12) and updating the position of said first markers as a function of these interactions; and
- updating (900) said three-dimensional model as a function of the updated position of the first markers and generating the images of the operation zone on the basis of the updated three-dimensional model.

2. A method according to claim 1, and comprising:
- creating (400) a virtual surgical instrument on the basis of the mechanical models of said surgical instrument (12);
- actuating (530) said virtual surgical instrument as a function of said movement commands; and determining (800), in real time, interactions between said operation zone and said surgical instrument (12) and updating the position of said first markers as a function of these interactions, comprising:
- calculating modifications of the operation zone and displacements of the virtual surgical instrument on the basis of the movement commands, of said three-dimensional model and of the mechanical models of the virtual surgical instrument; and
- calculating the updated position of said first markers as a function of said modifications to the operation zone.

3. A method according to claim 2, and comprising:
- calculating (600) feedback forces to be transmitted to said haptic device (11) as a function of said modifications of said operation zone and of said displacements of said virtual surgical instrument.

4. A method according to any one of the claims from 1 to 3, and comprising:
- saving (750), in said storage means (30), constraints referred to the patient's anatomy and/or optimal paths for said surgical instrument (12).

5. A method according to claim 1, and comprising:
- recording (430), by means of recording means (40) belonging to the apparatus (1), said three-dimensional model with second markers selected outside the patient's body.

6. A method according to claim 5, wherein updating (900) the three-dimensional model as a function of the updated position of the first markers comprises:
- updating, as a function of the position of the first markers, the three-dimensional model already recorded on the basis of the second markers.

7. A method according to claim 5 or 6, wherein said apparatus (1) comprises at least a second surgical robot device (21) provided with optical and/or ultrasound detection means (22) for acquiring in real time second data which allows a direct, real view of the operation zone; determining (800), in real time, interactions between said operation zone and said surgical instrument (12) and updating the position of said first markers as a function of these interactions, comprising:
- determining the updated position of said first markers and displacements of said surgical instrument (12) on the basis of said second data.

8. A method according to claim 7, and comprising:
- calculating (600) feedback forces to be transmited to said haptic device (11) as a function of a position and/or orientation error between the haptic device (11) and the surgical instrument (12), said position and/or orientation error being calculated as a function of said displacements of said surgical instrument (12).

9. An apparatus for minimally invasive surgical procedures comprising: storage means (30) for storing at least first data related to pre-surgery images of a patient's body zone to be subjected to a surgical operation; at least a first surgical robot device (21) provided with a respective surgical instrument (12); at least one haptic device (11) with force feedback to allow an operator to control said first surgical robot device (21); at least a second surgical robot device (12) provided with optical and/or ultrasound detection means (22) for acquiring in real time second data which allows a direct, real view of the operation zone; processing means (60); and software means stored in the storing means (30), loadable on the processing means (60) and designed to generate, when run on the processing means (60), images of the operation zone; said software means comprising:
- first software means (SWM) designed to create a three-dimensional, graphical-physical model of the operation zone on the basis of said first data and of a mathematical model calibrated on the mechanical features of the organs of the operation zone;
- second software means (SWMAIN) designed to acquire, by means of man-machine interface means of the apparatus (1), first markers of said three-dimensional model;
- third software means (SWU) designed to determine, in real time and on the basis of said second data, an updated position of said first markers; and
- fourth software means (SWS) for updating said three-dimensional model as a function of the updated position of the first markers and generating the images of the operation zone on the basis of the updated three-dimensional model.

10. An apparatus according to claim 9, and comprising recording means (40) for recording said three-dimensional model with second markers selected outside the patient's body; said fourth software means (SWS) being designed to update, as a function of the position of the first markers, the three-dimensional model previously recorded on the basis of the second markers.

11. An apparatus according to claim 9 or 10, wherein said third software means (SWS) are designed to determine, in real time and on the basis of said second data, the displacements of said surgical instrument (12); said software means comprising fifth software means (SWF) for calculating feedback forces to be transmitted to said haptic device (11) as a function of a position and/or orientation error between the haptic device (11) and the surgical instrument (12), said position and/or orientation error being calculated as a function of said displacements of the surgical instrument (12).

12. An apparatus according to any one of the claims from 9 to 11, wherein second software means (SWMAIN) are designed to acquire, by means of said haptic device (11), movement commands that the operator intends to provide to said first surgical robot device (21); said fourth software means (SWS) being designed to create a virtual surgical instrument on the basis of mechanical models of said surgical instrument (12), actuating said virtual surgical instrument as a function of said movement commands, calculating modifications of the operation zone on the basis of the movement commands, of said three-dimensional model and of the mechanical models of the virtual surgical instrument, and calculating the updated position of said first markers as a function of said modifications of the operation zone.

13. An apparatus according to claim 12, wherein said fourth software means (SWS) are designed to calculate displacements of the virtual surgical instrument on the basis of the movement commands, of said three-dimensional model and of the mechanical models of the virtual surgical instrument and calculating, as a function of said modifications of said operation zone and of said displacements of the virtual surgical instrument, feedback forces to be transmitted to said haptic device (11).

14. An apparatus according to any one of the claims from 9 to 13, wherein said software means comprise sixth software means (SWP) to save, in said storage means (30), constraints referred to the patient's anatomy and/or optimal paths for said surgical instrument (12).
